(19) 

Europäisches
Patentamt
European
Patent Office
Office européen
des brevets

(11) **EP 4 411 741 A1**

(12) **EUROPEAN PATENT APPLICATION**
published in accordance with Art. 153(4) EPC

(43) Date of publication:
**07.08.2024 Bulletin 2024/32**

(21) Application number: **22875579.9**

(22) Date of filing: **28.07.2022**

(51) International Patent Classification (IPC):
***G16B 40/00*** *(2019.01)*

(52) Cooperative Patent Classification (CPC):
**G16B 40/00**

(86) International application number:
**PCT/JP2022/029057**

(87) International publication number:
**WO 2023/053702 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2021 JP 2021159736**

(71) Applicant: **FUJIFILM Corporation
Tokyo 106-8620 (JP)**

(72) Inventors:
• **SHIMURA, Ryo
Ashigarakami-gun, Kanagawa 258-8538 (JP)**
• **GOTO, Kengo
Ashigarakami-gun, Kanagawa 258-8538 (JP)**
• **SATO, Masahiro
Ashigarakami-gun, Kanagawa 258-8538 (JP)**

(74) Representative: **Hughes, Andrea Michelle
Dehns Germany
Theresienstraße 6-8
80333 München (DE)**

(54) **DATA PROCESSING METHOD, DATA PROCESSING DEVICE, AND DATA PROCESSING PROGRAM**

(57) An embodiment according to the technology of the present disclosure provides a data processing method, a data processing apparatus, and a data processing program that can accurately extract a signal derived from a tumor cell from data in which a signal derived from a non-tumor cell is mixed. According to an aspect of the present invention, there is provided a data processing method executed by a data processing apparatus including a processor. The data processing method including causing the processor to execute: an input step of inputting first DNA profile data obtained by measuring a sample including a tumor cell and a plurality of known non-tumor cells; a signal removal step of removing a signal derived from the non-tumor cell, which is mixed in the input first DNA profile data, to acquire only a signal derived from the tumor cell; and an output step of outputting the signal derived from the tumor cell as a DNA profile feature amount of the sample.

FIG. 3

**Description**

**BACKGROUND OF THE INVENTION**

1. Field of the Invention

The present invention relates to a data processing method, a data processing apparatus, and a data processing program that extract a signal derived from a tumor cell from data in which a signal derived from a non-tumor cell is mixed.

2. Description of the Related Art

For example, JP2009-537827A discloses an apparatus and method for inspecting and/or removing a substance in a sample with fluorescence, regarding a technique for extracting a signal derived from a tumor cell from data in which a signal derived from a non-tumor cell is mixed. In addition, "A reference profile-free deconvolution method to infer cancer cell-intrinsic subtypes and tumor-type-specific stromal profiles", Li Wang et al., [Searched on September 22, 2021], Internet (https://genomemedicine.biomedcentral.com/track/pdf/10.1186/s13073-020-0720-0.pdf) discloses a reference-free signal decomposition technique considering the major classification of a cell that is likely to be mixed. "MethylResolver-a method for deconvoluting bulk DNA methylation profiles into known and unknown cell contents", Douglas Arneson et al., [Searched on September 22, 2021], Internet (https://www.nature.com/articles/s42003-020-01146-2.pdf) discloses that a reference is created from measurement data of sorted immune cells by a reference-based method.

**SUMMARY OF THE INVENTION**

An embodiment according to the technology of the present disclosure provides a data processing method, a data processing apparatus, and a data processing program that can accurately extract a signal derived from a tumor cell from data in which a signal derived from a non-tumor cell is mixed.

In order to achieve the above-described object, according to a first aspect of the present invention, there is provided a data processing method executed by a data processing apparatus including a processor. The data processing method comprises causing the processor to execute: an input step of inputting first DNA profile data obtained by measuring a sample including a tumor cell and one or more types of known non-tumor cells; a signal removal step of removing a signal derived from the non-tumor cell, which is mixed in the input first DNA profile data, to acquire a signal derived from the tumor cell; and an output step of outputting the signal derived from the tumor cell as a DNA profile feature amount of the sample.

According to a second aspect, in the data processing method according to the first aspect, the processor may be configured to acquire information that reflects features of a cell and/or a tissue defined by a sequence and/or modification of DNA as the first DNA profile data in the input step.

According to a third aspect, in the data processing method according to the second aspect, the processor may be configured to acquire, as the information, a measured value of at least one of a methylation state of DNA, mutation information of DNA, or a gene expression level.

According to a fourth aspect, in the data processing method according to any one of the first to third aspects, the processor may be configured to: input second DNA profile data that is different from the first DNA profile data and is sorted for each of known non-tumor cell types, which are likely to be mixed in the first DNA profile data, in the input step; and in the signal removal step, create a typical pattern matrix composed of typical patterns of the non-tumor cell types on the basis of the second DNA profile data, decompose the first DNA profile data into a signal for each of the non-tumor cell types using the first DNA profile data and the typical pattern matrix, remove a true residual from a residual of a result of the decomposition to calculate a residual corresponding to the signal derived from the tumor cell, and scale the calculated residual to acquire the signal derived from the tumor cell.

According to a fifth aspect, in the data processing method according to the fourth aspect, the processor may be configured to: in a case where M, N, and K are positive integers, receive N samples for M feature amounts as the second DNA profile data for K cell types; and create K types of M-dimensional typical pattern vectors from the received second DNA profile data and connect the K types of typical pattern vectors to create the typical pattern matrix of M rows and K columns.

According to a sixth aspect, in the data processing method according to the fourth or fifth aspect, the processor may be configured to perform the decomposition using a linear regression method.

According to a seventh aspect, in the data processing method according to the sixth aspect, the processor may be configured to use a least square method or a robust linear regression method as the linear regression method.

According to an eighth aspect, in the data processing method according to any one of the first to seventh aspects, the processor may be configured to perform the decomposition using a semi-reference-based method using some of the known typical pattern matrices.

According to a ninth aspect, in the data processing method according to the sixth or seventh aspect, the processor may be configured to: extract a residual of a result of regression performed by the linear regression method; and perform post-processing based on properties of the DNA profile feature amount on the extracted residual.

According to a tenth aspect, in the data processing method according to the ninth aspect, the processor may be configured to divide the calculated residual by an abundance ratio of a tumor in the input first DNA profile data to perform the scaling.

According to an eleventh aspect, in the data processing method according to any one of the fourth to tenth aspects, the processor may be configured to, in the signal removal step, factorize a matrix indicating the first DNA profile data into a mixing ratio matrix indicating a mixing ratio of cell types and a typical pattern matrix for the mixing ratio matrix to acquire the signal derived from the tumor cell and reconstruct the DNA profile feature amount from the acquired signal.

According to a twelfth aspect, in the data processing method according to the eleventh aspect, the processor may be configured to perform the matrix factorization using a singular value decomposition method or a non-negative matrix factorization method.

According to a thirteenth aspect, in the data processing method according to any one of the first to third aspects, the processor may be configured to, in the signal removal step, acquire the signal derived from the tumor cell using an abundance ratio of a tumor in the first DNA profile data which has been calculated by a method different from matrix factorization and reconstruct the DNA profile feature amount from the acquired signal.

According to a fourteenth aspect, in the data processing method according to the thirteenth aspect, the processor may be configured to acquire the signal derived from the tumor cell using a machine learning method.

According to a fifteenth aspect, in the data processing method according to any one of the eleventh to fourteenth aspects, the processor may be configured to: reconstruct the DNA profile feature amount of the sample including a component of the signal derived from the tumor cell, using a mixing ratio matrix corresponding to the acquired signal derived from the tumor cell; and divide the reconstructed DNA profile feature amount by the abundance ratio of the tumor in the DNA profile feature amount to perform scaling.

In order to achieve the above-described object, according to a sixteenth aspect of the present invention, there is provided a data processing apparatus comprising a processor. The processor is configured to execute: an input process of inputting first DNA profile data obtained by measuring a sample including a tumor cell and one or more types of known non-tumor cells; a signal removal process of removing a signal derived from the non-tumor cell, which is mixed in the input first DNA profile data, to acquire a signal derived from the tumor cell; and an output process of outputting the signal derived from the tumor cell as a DNA profile feature amount of the sample. The data processing apparatus according to the sixteenth aspect may have a configuration of executing the same processes as those in the second to fifteenth aspects.

In order to achieve the above-described object, according to a seventeenth aspect of the present invention, there is provided a data processing program causing a computer to execute a data processing method. The data processing method includes: an input step of inputting first DNA profile data obtained by measuring a sample including a tumor cell and one or more types of known non-tumor cells; a signal removal step of removing a signal derived from the non-tumor cell, which is mixed in the input first DNA profile data, to acquire a signal derived from the tumor cell; and an output step of outputting the signal derived from the tumor cell as a DNA profile feature amount of the sample. The data processing program according to the seventeenth aspect may have a configuration of executing the same processes as those in the second to fifteenth aspects. In addition, a non-transitory computer-readable recording medium storing the data processing program according to these aspects is also included in the scope of the present invention.

## BRIEF DESCRIPTION OF THE DRAWINGS

Fig. 1 is a diagram illustrating a schematic configuration of a data processing apparatus.
Fig. 2 is a diagram illustrating a schematic configuration of a processing unit.
Fig. 3 is a diagram illustrating an outline of data processing according to the present invention.
Fig. 4 is a diagram illustrating an aspect of data processing according to a first embodiment.
Figs. 5A and 5B are diagrams illustrating an example of calculation of a mixing ratio of non-tumor cells.
Fig. 6 is a diagram illustrating an aspect of signal decomposition by a semi-reference-based method.
Fig. 7 is a diagram conceptually illustrating results of the signal decomposition.
Fig. 8 is a diagram illustrating an aspect of data processing according to a second embodiment.
Fig. 9 is a diagram illustrating an example of reference-free signal decomposition.
Fig. 10 is a diagram illustrating another example of the reference-free signal decomposition.
Fig. 11 is a table illustrating results of a prediction experiment.

Fig. 12 is a diagram illustrating the results of the prediction experiment.

Figs. 13A and 13B are diagrams illustrating an example in which a change in the degree of methylation by a method according to the present invention visualized.

## DESCRIPTION OF THE PREFERRED EMBODIMENTS

[Extraction of Signal Derived from Tumor Cell]

In general, a signal derived from a cell type other than a tumor tissue is mixed in data obtained by measuring a gene expression level or methylation state of the tumor tissue. An example of one of the causes of this heterogeneity is that the tumor tissue is not capable of being accurately collected in a biopsy and surrounding cells are mixed. There is a concern that, due to the influence of the data heterogeneity, a tumor-derived signal desired to be originally detected will be buried in other signals and an expected result will not be obtained in the subsequent data analysis.

It is experimentally very expensive or difficult to sort a single cell type with the current technique. In addition, a tumor tissue measured in the past also has the feature of the data heterogeneity, and techniques for removing the data heterogeneity on computers have been studied in order to utilize the data already acquired.

The application of a signal decomposition technique is generally used as an approach to this problem. Signal decomposition is a technique that assumes typical patterns unique to each cell type and calculates the typical patterns and mixing ratios thereof such that measurement data is expressed by superimposition of the typical patterns (weighted by the mixing ratio and added).

The signal decomposition techniques according to the related art can be broadly classified into two types. One is a technique that optimizes only the mixing ratio on the basis of a reference matrix and is called a reference-based method. The reference matrix is a matrix created by acquiring typical patterns for each cell type in advance with any method. For example, it is assumed that immune cells are classified into a type A, a type B, and a type C and the types A to C are present in a sample at an unknown mixing ratio. In this case, it is assumed that each type can be sorted using a cell sorting technique and patterns $V_A = [1, 0, 0]^T$, $V_B = [0, 1, 0]^T$, and vc = $[0, 0, 1]^T$ for each type can be obtained. The reference matrix is a matrix $V = [V_A, V_B, V_C]$ in which column vectors are laterally arranged. A mixing ratio of [0.6, 0.1, 0.3] can be calculated from measurement data [0.6, 0.1, 0.3] by signal decomposition based on the reference matrix ($\because$[0.6, 0.1, 0.3] = [0.6 $\times$ $V_A$ + 0.1 $\times$ $V_B$ + 0.3 $\times$ vc]).

The creation of a tumor reference matrix is given as an example of the problem of the reference-based method. Unlike immune cells that can be sorted using a cell surface antigen, it is currently very difficult to sort tumors. Moreover, it is known that tumor cells have different cell features depending on the cells even in the same tumor tissue, which is referred to as "intra-tumor heterogeneity". Therefore, in a case where tumor data is targeted, it is difficult to obtain an expected effect directly by the reference-based method.

In that respect, a reference-free method has been developed as another signal decomposition technique that does not require the reference matrix. This is a technique that optimizes patterns for each cell type which is treated as the reference matrix at the same time as the mixing ratio. A method has also been developed which can be applied to data from which the reference matrix is difficult to obtain in advance, such as tumor data, and is specialized for the tumor data ("A reference profile-free deconvolution method to infer cancer cell-intrinsic subtypes and tumor-type-specific stromal profiles", Li Wang et al., [Searched on September 22, 2021], Internet (https://genomemedicine.biomedcentral.com/track/pdf/10.1186/s13073-020-0720-0.pdf)).

On the other hand, the method has a problem in that there are many variables to be optimized and many samples are required for accuracy. Further, the reference-free method also requires a process of mapping which cell type the calculated pattern is derived from.

A method that allows the mixture of tumors and performs reference-based decomposition has been developed in order to solve the problem that "it is difficult to create a tumor reference matrix" ("MethylResolver-a method for deconvoluting bulk DNA methylation profiles into known and unknown cell contents", Douglas Arneson et al., [Searched on September 22, 2021], Internet (https://www.nature.com/articles/s42003-020-01146-2.pdf)). This method is characterized in that an immune cell reference which is easily mixed into a tumor sample and can be sorted is created, reference-based decomposition is performed, and signals other than the reference pattern are decomposed by a method capable of reducing the influence of the mixture. Therefore, it is possible to estimate the mixing ratio of non-tumor cells mixed into the tumor sample. As a result, it is possible to estimate an abundance ratio of tumors with high accuracy.

However, so far, no techniques for accurately extracting signals derived from tumor cells have been developed. The reference-based method according to the related art is inappropriate because it requires a tumor reference in the first place. Even in "MethylResolver-a method for deconvoluting bulk DNA methylation profiles into known and unknown cell contents", Douglas Arneson et al., [Searched on September 22, 2021], Internet (https://www.nature.com/articles/s42003-020-01146-2.pdf), it is possible to estimate the abundance ratio, but it is not possible to extract the signal pattern. The reference-free method has been so far applied to the signal decomposition of the tumor sample. However, there is no case where the reference-free method is used for the tumor sample in combination with a process for reducing the influence of the mixture of non-cancer cells.

The inventors of the present invention conducted intensive studies under these circumstances and obtained an idea for a data processing method, a data processing apparatus, and a data processing program capable of accurately extracting signals derived from tumor cells from data mixed with signals derived from non-tumor cells. Hereinafter, embodiments of the present invention will be described. In the description, the accompanying drawings will be referred to as necessary. In addition, in the accompanying drawings, some components may be omitted for convenience of description.

[Configuration of Data Processing Apparatus]

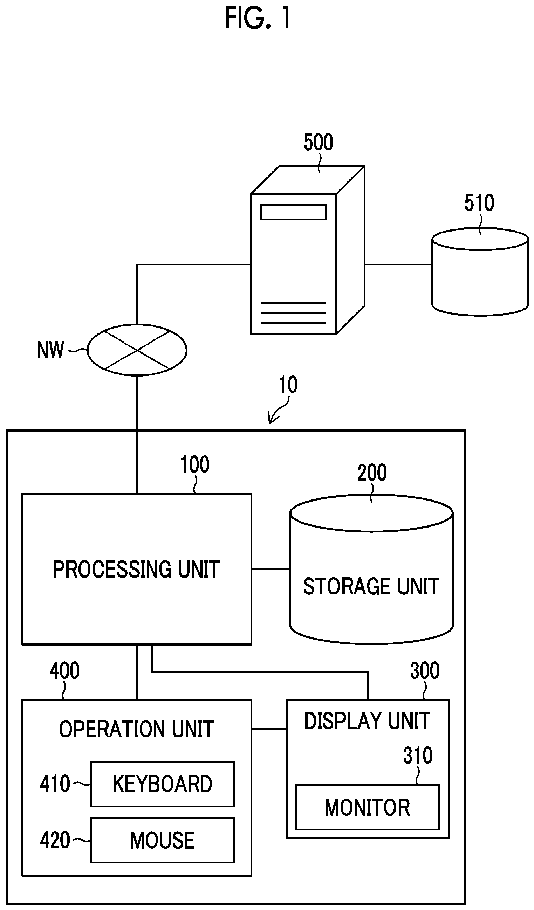

Fig. 1 is a diagram illustrating a schematic configuration of a data processing apparatus according to an embodiment of the present invention. As illustrated in Fig. 1, a data processing apparatus 10 (data processing apparatus) according to the first embodiment comprises a processing unit 100 (a processor or a computer), a storage unit 200, a display unit 300, and an operation unit 400. These components are connected to one another to transmit and receive necessary information. Various installation forms can be adopted for these components. These components may be installed in one place (in one housing, one room, or the like) or may be installed in places separated from each other and connected via a network. In addition, the data processing apparatus 10 is connected to an external server 500 and/or an external database 510 via a network NW, such as the Internet, can acquire samples, learning data, and the like (for example, TCGA data which will be described below) used for data processing as necessary, and can store processing results and the like in the external server 500 and/or the external database 510.

[Configuration of Processing Unit]

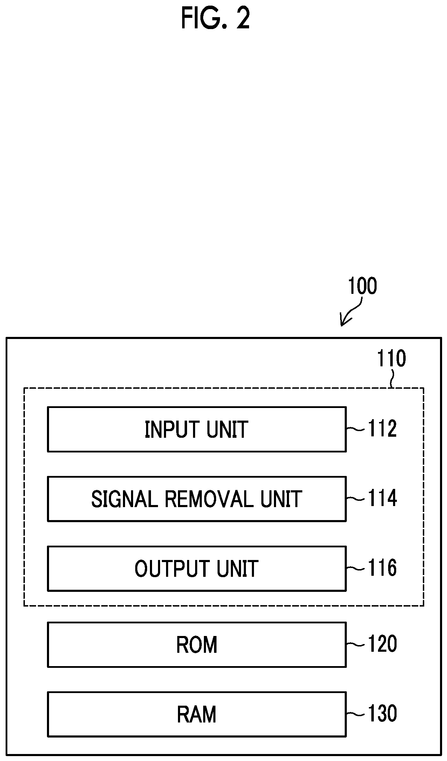

As illustrated in Fig. 2, the processing unit 100 comprises a processor 110 (a processor or a computer), a read only memory (ROM) 120, and a random access memory (RAM) 130. The processor 110 performs the overall control of processes performed by each unit of the processing unit 100 and has functions of an input unit 112, a signal removal unit 114, and an output unit 116. An outline of these functions will be described. The input unit 112 performs an input step (input process) of inputting first DNA profile data obtained by measuring a sample including a tumor cell and a plurality of known non-tumor cells. The signal removal unit 114 performs a signal removal step (signal removal process) of removing signals derived from the non-tumor cells mixed in the input first DNA profile data (DNA) and acquiring a signal derived from the tumor cell. The output unit 116 performs an output step (output process) of outputting the signal derived from the tumor cell as a DNA profile feature amount of the sample. The input unit 112 can receive an input via the network NW or a recording medium, such as the storage unit 200, and the output unit 116 can perform display on a monitor 310 and output to the storage unit 200.

The functions of each unit of the processing unit 100 and the processor 110 can be implemented by various processors and a recording medium. The various processors include, for example, a central processing unit (CPU) which is a general-purpose processor that executes software (program) to implement various functions. In addition, the various processors also include a graphics processing unit (GPU), which is a processor specialized for image processing, and a programmable logic device (PLD), which is a processor whose circuit configuration can be changed after manufacturing, such as a field programmable gate array (FPGA). In a case where learning or recognition of images is performed, the configuration using the GPU is effective. Further, the various processors also include a dedicated electric circuit which is a processor having a dedicated circuit configuration designed to perform a specific process such as an application specific integrated circuit (ASIC).

The functions of each unit may be implemented by one processor or may be implemented by a plurality of processors of the same type or different types (for example, a plurality of FPGAs, a combination of the CPU and the FPGA, or a combination of the CPU and the GPU). In addition, a plurality of functions may be implemented by one processor. A first example of the configuration in which the plurality of functions are configured by one processor is an aspect in which one processor is configured by a combination of one or more CPUs and software and implements the plurality of functions. A representative example of this aspect is a computer. A second example of the configuration is an aspect in which a processor that implements the functions of the entire system using one integrated circuit (IC) chip is used. A representative example of this aspect is a system-on-chip (SoC). As described above, various functions are configured using one or more of the above-described various processors as a hardware structure. In addition, specifically, the hardware structure of the various processors is an electric circuit (circuitry) obtained by combining circuit elements such as semiconductor elements. The electric circuit may be an electric circuit that implements the above-described functions using a logical sum, a logical product, a logical negation, an exclusive logical sum, and a logical operation of a combination thereof.

In a case where the processor or the electric circuit executes software (program), codes that can be read by a computer (for example, various processors or electric circuits constituting the processing unit 100 and/or a combination thereof) of the executed software are stored in a non-transitory recording medium, such as the ROM 120, and the computer refers to the software. The software stored in the non-transitory recording medium includes a program (data processing program) for executing the data processing method according to the embodiment of the present invention and data (first and second DNA profile data items, learning data, such as cancer type labels which will be described below, weight parameters used in machine learning, and the like) used in a case of execution. The codes may be recorded on non-transitory recording media, such as various magneto-optical recording devices and semiconductor memories, instead of the ROM 120. In a case of processes using software, for example, the RAM 130 is used as a transitory storage area. In addition, data stored in a non-transitory recording medium (not illustrated), such as an electronically erasable and programmable read only memory (EEPROM) or a flash memory, can also be referred to. The storage unit 200 may be used as the "non-transitory recording medium".

Details of a process using the processing unit 100 having the above-described configuration will be described below.

<Configuration of Storage Unit>

The storage unit 200 is composed of various storage devices, such as a hard disk and a semiconductor memory, and a control unit therefor and can store the first and second DNA profile data items, the learning data, such as cancer type labels, the weight parameters used in machine learning, execution results of the data processing method, and the like.

<Configuration of Display Unit>

The display unit 300 comprises the monitor 310 (display device) that is configured by a display, such as a liquid crystal display, and can display input data, the execution results of the data processing method/data processing program, and the like. The monitor 310 may be configured by a touch panel display and may receive the input of an instruction from a user.

<Configuration of Operation Unit>

The operation unit 400 comprises a keyboard 410 and a mouse 420, and the user can perform operations related to the execution of the data processing method according to the embodiment of the present invention, the display of results, and the like through the operation unit 400. The operation unit 400 may comprise other operation devices.

[Outline of Data Processing]

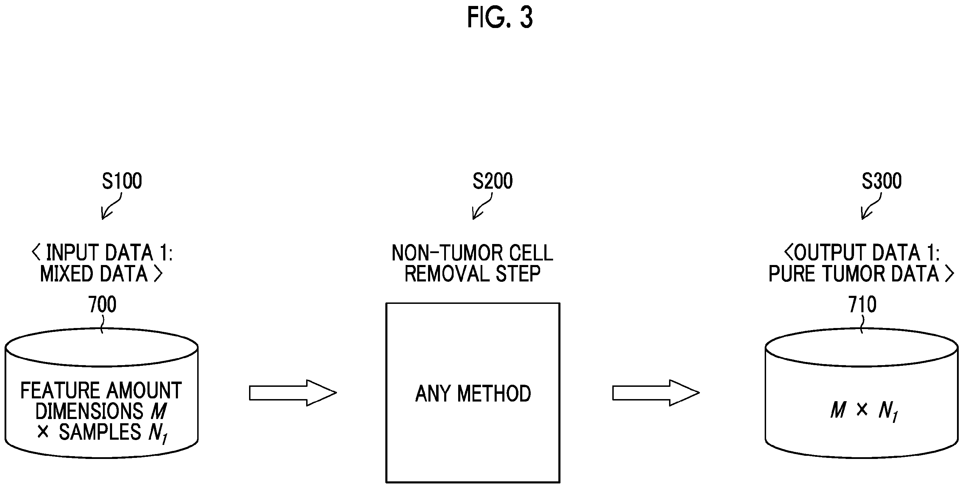

Fig. 3 is a diagram illustrating an outline of data processing according to the embodiment of the present invention. The input unit 112 (processor) inputs first input data 700 (first DNA profile data) obtained by measuring a sample including a tumor cell and one or more types of known non-tumor cells (Step S100: an input step, and an input process). The first input data 700 is matrix data (a matrix of M rows $\times$ N1 columns) consisting of M feature amount dimensions $\times$ N1 samples, and the input unit 112 can acquire, as the first input data 700 (the first DNA profile data), information which reflects the features of cells and/or tissues defined by DNA sequences and/or modifications, specifically, for example, a measured value of at least any of a methylation state of DNA, mutation information of DNA, or a gene expression level.

Further, in the first embodiment and a second embodiment which will be described below, K, M, N, N1, N2, and N' are any positive integers (natural numbers equal to or greater than 1).

The signal removal unit 114 (processor) removes a signal derived from the non-tumor cell from the first input data 700 using various methods including a method, which will be described below, to acquire a signal derived from the tumor cell (Step S200: a signal removal step, and a signal removal process), and the output unit 116 (processor) outputs the acquired signal as a DNA profile feature amount of the sample (Step S300: an output step, and an output process). Output data 710 (the DNA profile feature amount of the sample) is a matrix of M rows $\times$ N1 columns, similarly to the first input data 700.

[First Embodiment of Signal Removal]

In a first embodiment of the signal removal, the signal removal is performed using a reference for the non-tumor cell. A reference for the tumor cell is not used. Hereinafter, the signal removal in Step S200 will be mainly described in detail.

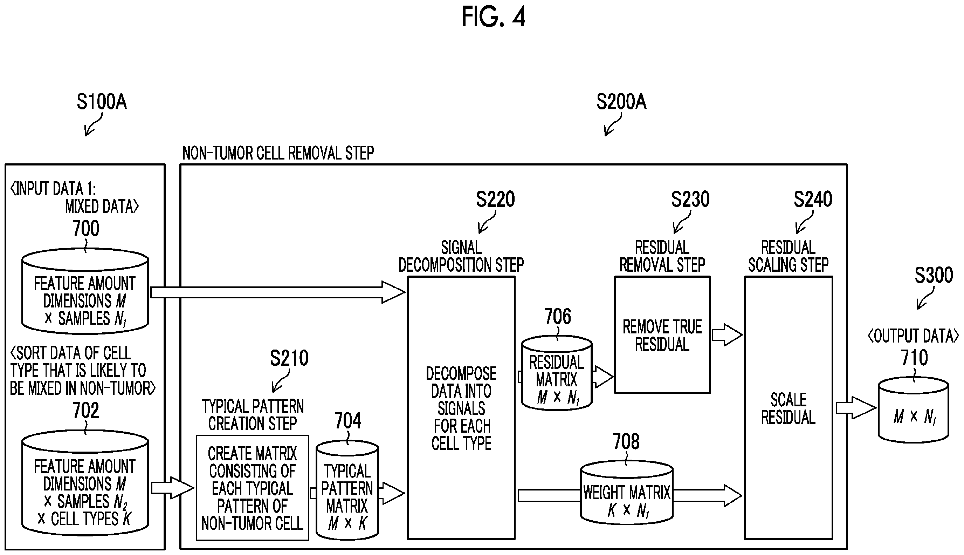

Fig. 4 is a diagram illustrating an aspect of the data processing according to the first embodiment. The input unit 112 (processor) inputs second input data 702 (second DNA profile data) that is different from the first input data 700 (first DNA profile data) and that is sorted for each of known non-tumor cell types which are likely to be mixed in the first input data 700 (Step S100A: an input step, and an input process). Similarly to the first input data 700, the second input data 702 is information which reflects the features of cells and/or tissues defined by DNA sequences and/or modifications, specifically, for example, a measured value of at least one of a methylation state of DNA, mutation information of DNA, or a gene expression level and is a matrix of M rows × N2 columns for K cell types.

A process in the non-tumor cell removal step (non-tumor cell removal process) of Step S200A will be described in detail. The signal removal unit 114 (processor) creates K types of typical pattern matrices 704 (typical pattern matrices) from the second input data 702 (second profile data) using a typical pattern creation unit (processor) that is included in the signal removal unit 114 (Step S210: a typical pattern creation step/a typical pattern creation process, a non-tumor cell removal step/a non-tumor cell removal process, and a signal removal step/a signal removal process). For example, the typical pattern creation unit sets a vector that has an intermediate value between the samples of the same cell type as a typical pattern vector (M-dimensional vector) of the cell type and connects the created K types of typical pattern vectors to form one matrix (= a reference matrix B). The typical pattern matrix 704 is a matrix of M rows × K columns (in a case where the second input data 702 is a matrix of M rows × N columns, the typical pattern matrix 704 is a matrix of M rows × K columns). In addition, the typical pattern creation unit may calculate representative values, such as an average value and a mode value, other than the intermediate value between the samples of the same cell type for each element with any method to create the typical pattern matrix.

Then, the signal removal unit 114 (processor) regresses the input data using the reference matrix (typical pattern matrix) (Step S220: a signal decomposition step, and a signal decomposition process). That is, the signal removal unit 114 calculates a coefficient matrix F (mixing ratio matrix: a matrix of K rows × N1 columns) indicating the mixing ratio where the following (Equation 1) is established. That is, the typical pattern matrix 704 (reference matrix B) is an explanatory variable, and a matrix X indicating the measured value is the explanatory variable.

$$X = BF + R \quad \text{(Equation 1)}$$

In (Equation 1), an i-th row of the coefficient matrix F indicates an estimated mixing ratio of a sample i, and R indicates a residual term.

In a general linear regression method, the coefficient matrix F can be calculated by a least square method (LS method) as represented by the following (Equation 2).

$$\hat{F} = \left( B^{\mathrm{T}} B \right)^{-1} B^{\mathrm{T}} X \quad \text{(Equation 2)}$$

Further, in a case where a robust linear regression method is used, for example, in the case of a least trimmed squares method (LTS method: see Rousseeuw, P. J.; Leroy, A. M. (2005) [1987]. Robust Regression and Outlier Detection. Wiley. doi:10.1002/0471725382. ISBN 978-0-471-85233-9. and the like), the signal removal unit 114 alternately and repeatedly performs the selection of a subset that minimizes the square residual from M feature amount sets and the ordinary least square method. That is, the signal removal unit 114 calculates the matrix F using the subset S satisfying the following (Equation 3) and the LS method using the feature amount subset at that time.

$$\min_{S} \sum_{j \in S} r_{(j)}^2 \quad \text{(Equation 3)}$$

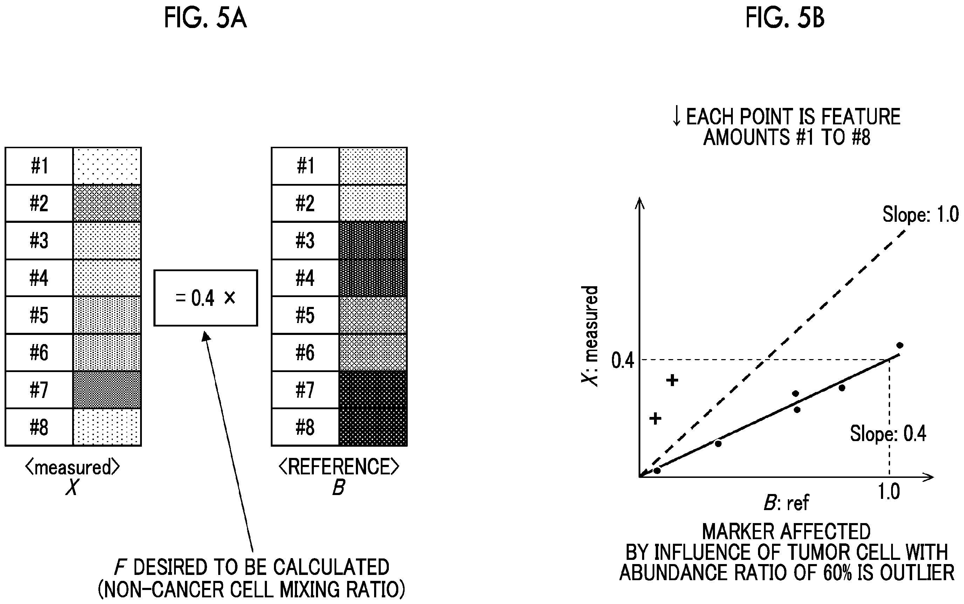

Figs. 5A and 5B are diagrams each of which illustrates an example of the calculation of the non-tumor cell mixing ratio in a case where M (the number of feature amounts) = 8, N1 = 1, and K = 1 are established. In this case, as illustrated in Fig. 5A, since N1 (the number of samples) = 1 and K (the number of cell types assumed to be mixed) = 1 are established, this is a problem to find a scalar F from one-dimensional vectors X and B. In other words, for X which is data obtained by measuring one cancer sample, in a case where a non-cancer cell reference B (typical pattern) assumed to be mixed in the sample is known, the mixing ratio (scalar F) of the non-cancer cell is calculated by linear regression.

First, the signal removal unit 114 performs the LS method on all of the feature amounts (from #1 to #8). It can be seen that the result is a dashed straight line in Fig. 5B and is a regression line which deviates from normal data represented by circles. In a case where the LTS method is applied, a subset having the minimum square residual is searched for further as illustrated in (Equation 3). In Fig. 5B, a subset of only the circles is specified except for outliers represented by crosses. It can be seen that a solid line is a regression line by the LTS method and is fitted to the circles well. further, in the case of Figs. 5A and 5B, since the mixing ratio of the non-cancer cells (scalar F) is 0.4, 40% of cells is non-cancer cells (60% of cancer cells), and this is often seen in actual situations such as data from TCGA (GDC Data Portal: see https://portal.gdc.cancer.gov/).

In practice, the vector X (measurement data) has columns corresponding to "the number of samples", and the reference B has columns corresponding to "the assumed number of cell types". Therefore, the feature amounts are not fitted to a straight line, but are fitted to a hyperplane. In addition, the scalar F (mixing ratio) is also a vector, and the mixing ratio for each cell type corresponds to each element.

[Decomposition by Semi-Reference-Based Method]

In addition, for the decomposition of the first input data 700 (first DNA profile data), a reference obtained by extracting only the feature amounts effective in the decomposition of the cell type may be used. That is, the signal removal unit 114 may perform the decomposition using a semi-reference-based method using some of the known typical pattern matrices. For example, a situation is assumed in which "there are 100 feature amounts and the values of 70 feature amounts do not change in the assumed cell types". In this case, it is not possible to distinguish the cell types with the 70 feature amounts. Therefore, it is not necessary to use the 70 feature amounts. Therefore, it is possible to perform the decomposition with a focus only on the remaining 30 feature amounts.

Here, the "semi-reference-based method" refers to a method having any one or two or more of the following features (1) to (3):

(1) A design is made such that it is not necessary to provide references for all of the components to be mixed, and the reference is used for a known component and is not used for an unknown component for a system in which unknown (non-reference) components are mixed;
(2) A design is made such that a reliability degree is defined in advance for references, and an error of a reference having a low reliability degree in a regression process is allowed; and
(3) A design is made such that a reference is not positively (directly) provided for the assumed mixed component, and some prior knowledge used for decomposition is given to the assumed mixed component in a different form, for example, by a distribution of possible values.

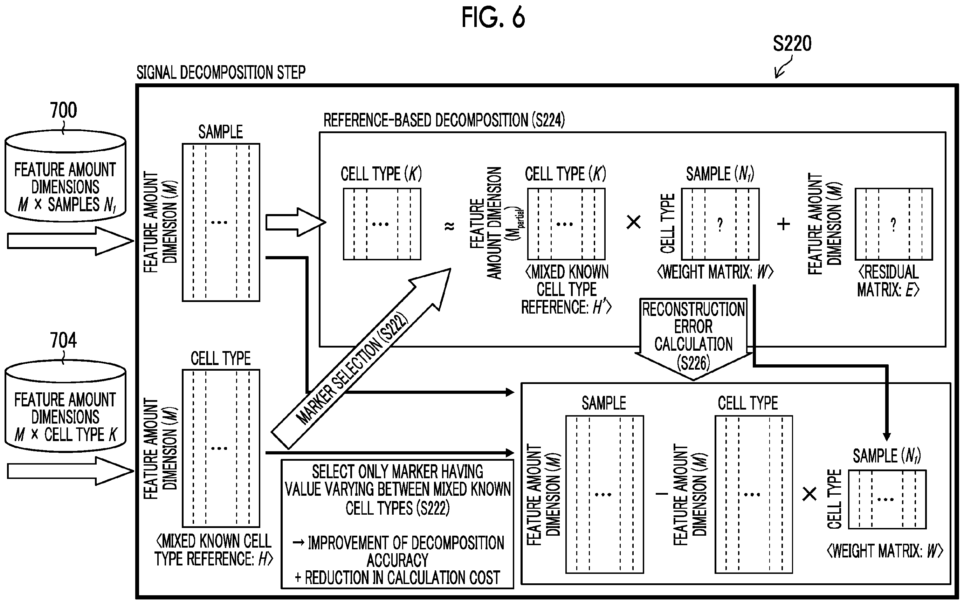

Fig. 6 is a diagram illustrating an aspect of signal decomposition by the semi-reference-based method and is a diagram illustrating in detail a portion of the signal decomposition step (signal decomposition process) of Step S220 in Fig. 4. The signal removal unit 114 selects some markers (feature amounts) whose values vary between the cell types (Step S222: a marker selection step/process, and a signal decomposition step/process), performs signal decomposition using the typical pattern matrix for the selected markers (Step S224: a signal decomposition step, and a signal decomposition process), and performs the reconstruction of the typical pattern matrix and the calculation of an error (Step S224: a signal decomposition step, and a signal decomposition process).

A calculation cost can be significantly saved and an increase in processing speed is expected by the decomposition using the semi-reference-based method. In addition, the feature amount that is noise in the decomposition is removed, and accuracy is also expected to be improved. In practice, in the case of methylation data, currently, a measurement platform having 450,000 feature amounts is the mainstream. However, immune cells can be distinguished by about 500 feature amounts, which is disclosed in "MethylResolver-a method for deconvoluting bulk DNA methylation profiles into known and unknown cell contents", Douglas Arneson et al., [Searched on September 22, 2021], Internet (https://www.nature.com /articles/s42003-020-01146-2.pdf).

[Results of Signal Decomposition]

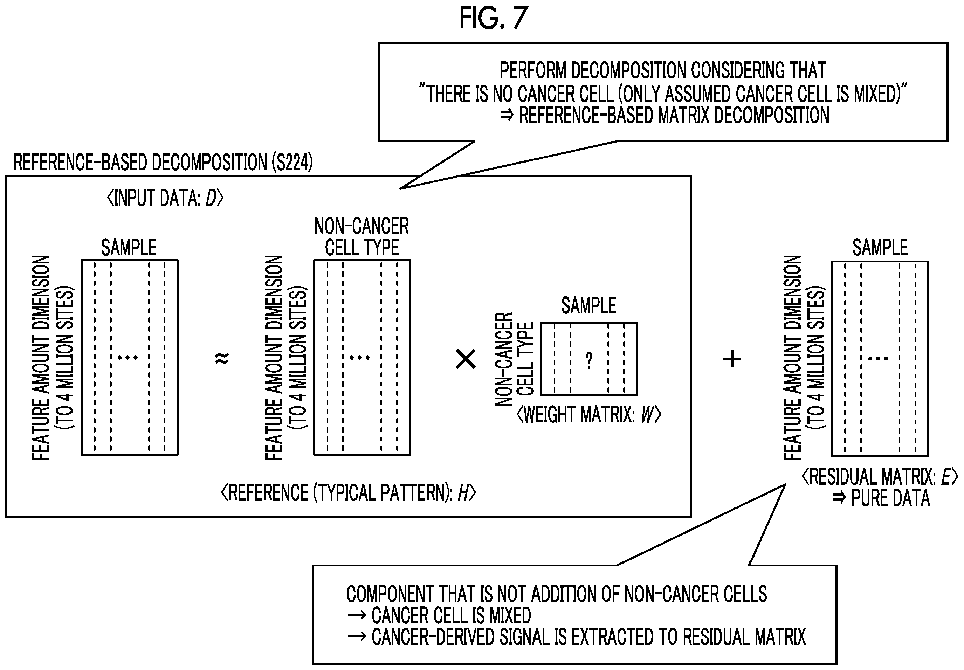

Fig. 7 is a diagram conceptually illustrating results of the signal decomposition. It is expected that, in the residual matrix calculated by the above-described signal decomposition (reference-based or semi-reference-based), the non-tumor component included in the reference (typical pattern) will be removed and tumor components will be extracted.

[Post-Processing for Residual]

However, the residual matrix also includes the original error associated with linear regression. Therefore, post-processing based on the properties of the input DNA profile feature amount (first input data 700) is performed. For example, in the case of methylation data, the measured value thereof is defined in a range of 0 or more and 1 or less. Therefore, the signal removal unit 114 (processor) performs a process of rounding off a value out of the range.

In a case where the residual matrix R is regarded as a pure tumor signal component from which the non-tumor component has been removed, an absolute value thereof depends on the abundance ratio of the tumor included in the original sample. For example, it is assumed that a true value of only the tumor of the sample A having a certain feature amount is 1.0, a true value of only the tumor of the sample B is 0.5, and a true value of the non-tumor mixed is 0.3. In this case, assuming that the abundance ratio of the tumor in A is 0.5 and the abundance ratio of the tumor in B is 1.0, the measured value in A is about 0.65 (= $1.0 \times 0.5 + 0.3 \times 0.5$), and the measured value in B is about 0.5 (= $0.5 \times 1.0 + 0.3 \times 0.0$).

In a case where these samples are subjected to a cleansing process (the removal of the signal derived from the non-tumor cell), a residual (= corresponding to a pure tumor signal) of 0.5 is ideally obtained in both the samples A and B. This is a value obtained by a multiplication of the abundance ratio of the tumor as described above. Therefore, conversely, the value of only the tumor can be obtained by dividing the value by the abundance ratio of the tumor. For example, for the sample A, 1.0 (the value of only the tumor) = 0.5 (the residual for the sample A) ÷ 0.5 (the abundance ratio of the tumor in the sample A) is established. In addition, for the sample B, 0.5 (the value of only the tumor) = 0.5 (the residual for the sample B) ÷ 1.0 (the abundance ratio of the tumor in the sample B) is established.

As a method for estimating the abundance ratio of the tumor, for example, the following known technique may be used: "ABSOLUTE" (see "Absolute quantification of somatic DNA alterations in human cancer" S. L. Carter, et al., 2012:https://dash.harvard.edu/handle/1/15034760); and "ESTIMATE" (see "Inferring tumour purity and stromal and immune cell admixture from expression data" K. Yoshihara, et al., 2013:https://www.nature.com/articles/ncomms3612.pdf). However, additional data is required to estimate the abundance ratio of the tumor. In a case where it is not possible to prepare additional data, it is possible to estimate the abundance ratio of the tumor on the basis of the sum of the mixing ratios of the results of the signal decomposition. For example, the abundance ratio of the tumor for any sample can be estimated by learning conversion from the sum of the mixing ratios to the estimated value of "ABSOLITE" in advance.

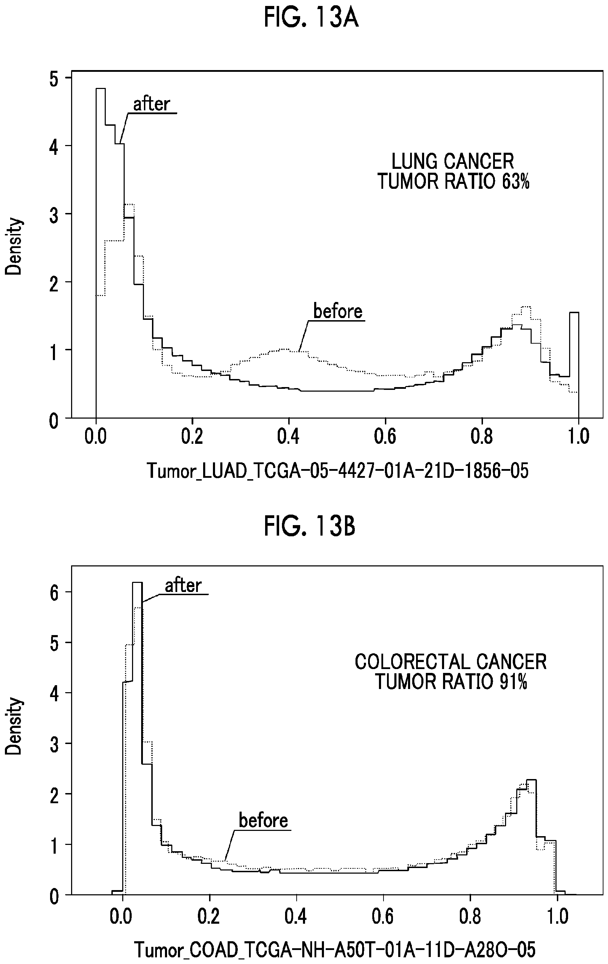

Further, it is also possible to perform scaling without using the abundance ratio of the tumor. For example, in the case of data in a methylation state, the value thereof is in the range of 0 or more and 1 or less as described above. The distribution of the methylation state has peaks in the vicinity of 0 and in the vicinity of 1. It is assumed that a distribution after the removal of the non-tumor component is the same as described above. Therefore, scaling can be performed by performing enlargement, reduction, and parallel translation such that two peaks are matched with 0 and 1 (see a visualization example in Examples described below; Fig. 13).

[Output of DNA Profile Feature Amount]

The output unit 116 (processor) outputs the signal derived from the tumor cell, which has been acquired by the above-described method, as the output data 710 (the DNA profile feature amount of the sample) (Step S300 in Figs. 3 and 4: an output step, and an output process).

According to the first embodiment described above, it is possible to accurately extract the signal derived from the tumor cell from data in which the signal derived from the non-tumor cell is mixed.

[Second Embodiment of Signal Removal]

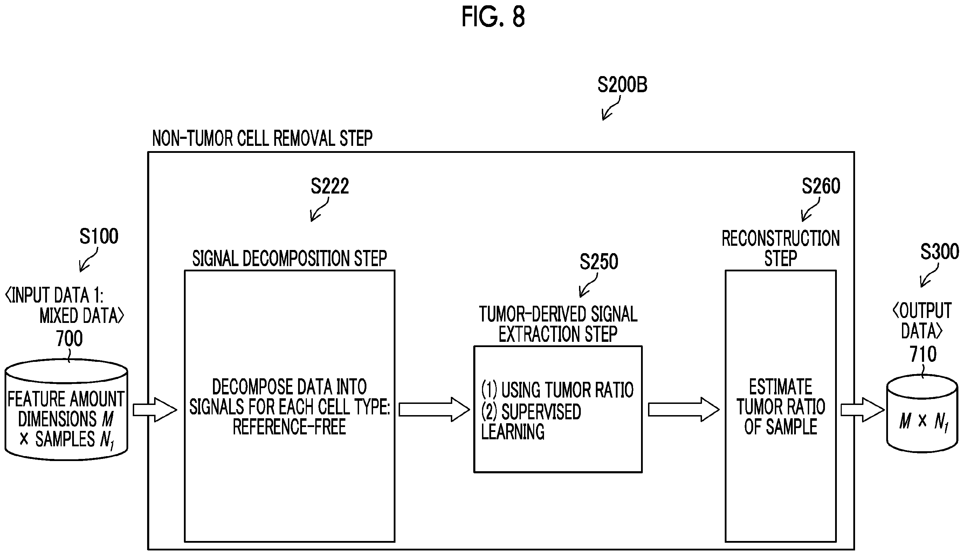

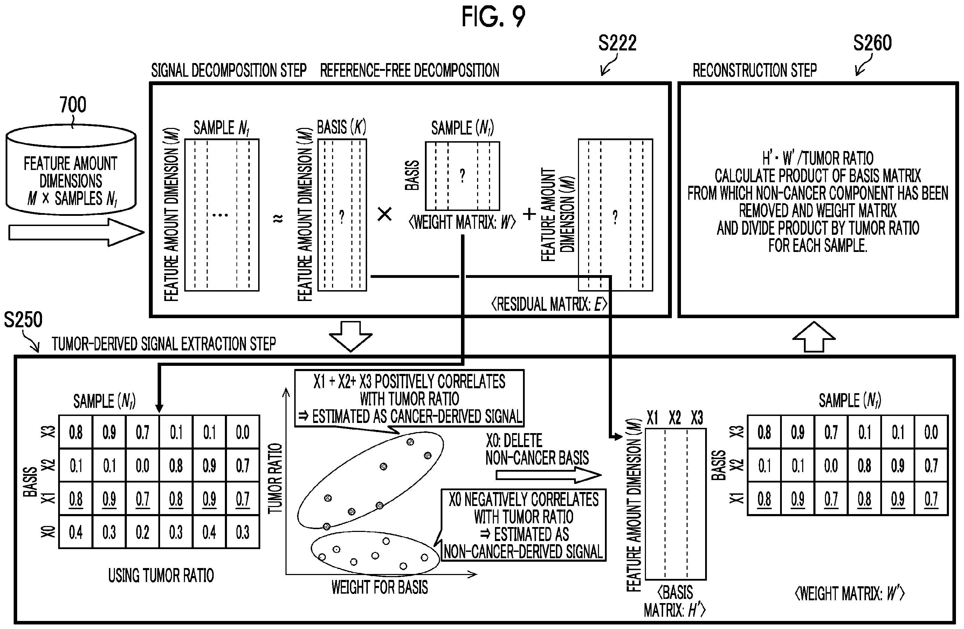

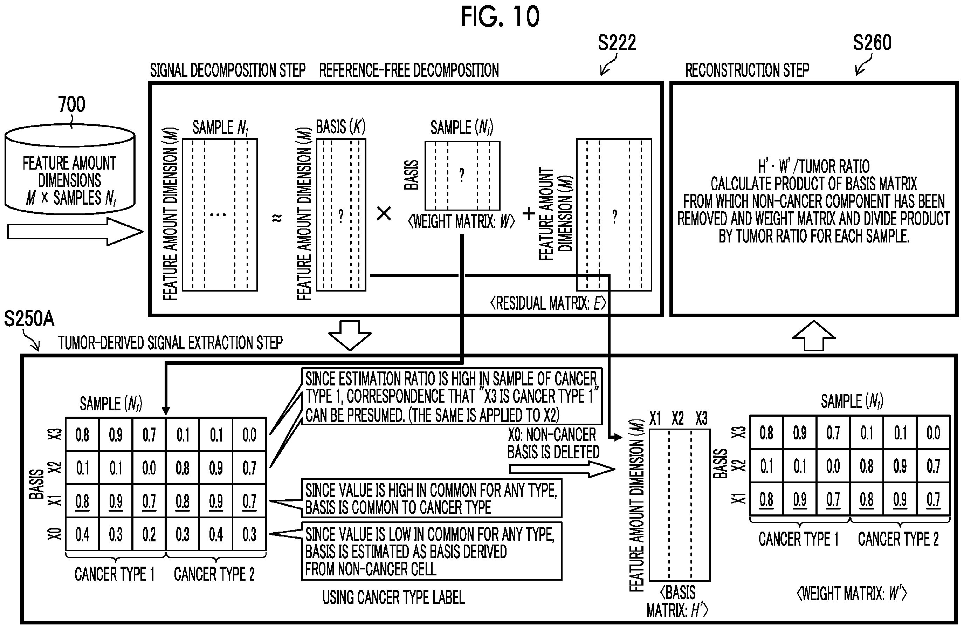

Next, a second embodiment of the signal removal will be described. In a method according to the second embodiment, the extraction of the signal derived from the tumor cell is performed in a reference-free manner. In the section "Extraction of Signal Derived from Tumor Cell", it has been stated that "since the reference-free method does not require pure tumor data in advance, it can be applied to data in which the non-cancer cell is mixed, and the performance thereof is an issue". However, the reference-free method can be applied to the present invention, and embodiments in this case will be described below. Even in the reference-free method, there is no case in which the results of signal decomposition are used to reduce the influence of the mixture of the non-cancer cell in the subsequent process. Hereinafter, a procedure will be described mainly with reference to Fig. 8 (a diagram illustrating an aspect of data processing in the second embodiment) and Fig. 9 (a diagram illustrating an example of reference-free signal decomposition). Fig. 10 (a diagram illustrating another example of the reference-free signal decomposition) is a variation of Fig. 9.

The input unit 112 (processor) inputs the first input data 700 (first DNA profile data) (Step S100: an input step, and an input process). The first input data 700 is a matrix of feature amount dimensions M × the number of samples N1. In the first and second embodiments, the dimensions of the feature amounts in the first input data 700 may be the same or different.

In the second embodiment, in a non-tumor cell removal step (non-tumor cell removal process) of Step S200B, the signal removal unit 114 (processor) decomposes the first input data 700 into signals for each cell type using the reference-free method (Step S222 in Figs. 8 to 10: a signal decomposition step, and a signal decomposition process). The reference-free method is a method that calculates potential feature patterns (bases) in the input data and a weight for determining "how to add the feature patterns". For example, in a system in which cells are mixed, each basis corresponds to each cell type. That is, a typical feature amount pattern of a certain cell type is referred to as the basis.

However, depending on the situation, there may be a basis corresponding to a subtype (a classification obtained by further subdividing one cell type) of the cell type. Therefore, the number of bases is not necessarily matched with the number of cell types.

As a known technique that performs signal decomposition using the reference-free method, singular value decomposition (SVD) is the most basic. In addition, non-negative matrix factorization (NMF) which imposes the restrictions that all elements of a basis matrix and a weight matrix are non-negative values is preferable in the cell profile data, and an application method based on NMF (various restrictions considering the properties of measurement data have been proposed in addition to the non-negativity) is more preferable. In addition, cleansing can be performed by the present invention even in a case where any matrix factorization algorithm that factorizes a matrix of M rows × N1 columns into a matrix of M rows × K columns and a matrix of K rows × N1 columns is used. However, which algorithm is suitable depends on input data. The signal removal unit 114 may determine the algorithm to be used according to the features of data or on the basis of the operation of the user through the operation unit 400.

The signal removal unit 114 specifies the basis derived from the cancer cell among the bases estimated as results of the matrix factorization and removes the basis derived from the non-cancer cell (Step S250: a tumor-derived signal extraction step, and a tumor-derived signal extraction process). The method illustrated in Fig. 9 is a method using the abundance ratio of the tumor in each sample (first DNA profile data) estimated by a method different from the matrix factorization. In the matrix in Step S250 of Fig. 9, each column indicates weights for the bases (X0 to X3) of the sample. For example, it is shown that measurement data in the first column of the sample can be expressed by $0.4 \times X0 + 0.8 \times X1 + 0.1 \times X2 + 0.8 \times X3$.

In this case, it is considered that the bases X0 to X3 correspond to, for example, cancer types or cell types, and it is desired to remove the basis derived from the non-cancer cell. Therefore, the signal removal unit 114 can examine the correlation between a tumor ratio and a weight for each basis to specify the basis derived from the non-cancer cell. In a simple example, it is assumed that only two cells of a cancer cell and a non-cancer cell are mixed and the basis X is the basis of the cancer, that is, "a typical pattern of the cancer cell". In this case, it can be seen that a weight for the basis X has a high correlation with the tumor ratio. In practice, it is also considered that the basis does not need to be a single basis and a combination of a plurality of bases (bases X1, X2, and X3 in the example illustrated in Fig. 9) correlates with the tumor ratio as illustrated in the example of Fig. 9. On the contrary, a weight for the basis (basis X0 in the example illustrated Fig. 9) to be removed is expected to have a negative correlation with the tumor ratio, and the signal removal unit 114 extracts a basis matrix H' and a weight matrix W' from which the basis has been removed.

In a case where a correct answer label, such as a cell type name, cancer, or non-cancer, is given to the sample, the signal removal unit 114 can remove the basis derived from the non-cancer cell (acquire the signal derived from the tumor cell) on the basis of the correct answer label using a machine learning method. Fig. 10 illustrates an example in which the left half of the sample is "cancer type 1" and the right half of the sample is "cancer type 2" (see Step S250A). In this case, weights for the bases X2 and X3 specific to the cancer type are increased, and the signal removal unit 114 can estimate that the bases X2 and X3 correspond to cancer type 1 and cancer type 2, respectively. In addition, since the basis X1 is not specific to the cancer type, but has a high value in all of the cancer samples. The signal removal unit 114 can estimate that the basis X1 is a basis common to cancer. The remaining basis X0 has a low weight in common for all of the samples. Therefore, the signal removal unit 114 can estimate that the basis X0 is a basis derived from the non-cancer cell.

As a machine learning method for removing the basis derived from the non-cancer cell, a method for supervised learning of qualitative labels can be used to learn the weights or degrees of importance of variables, and the weights and the degrees of importance can be used. For example, these are weights for variables in logistic linear regression, particularly, sparse logistic linear regression and the degree of importance of each variable obtained in random forest learning. In any case, it is possible to simply take a larger weight and a high degree of importance which are equal to or greater than threshold values among the obtained weights or degrees of importance of the variables. In addition, more advanced methods using known techniques have also been proposed. However, the basic idea is to "use weights for importance variables estimated by a trained model".

In addition, the "variable" described herein is the mixing ratio of the bases obtained as a result of the matrix factorization. For example, in a case where a certain sample is a cancer type X in which a basis A is 10%, a basis B is 5%, and a basis C is 70%, learning is performed such that "[10%, 5%, 70%] is input to correctly estimate X". The signal removal unit 114 (processor) may comprise a learning device that can perform this machine learning or a trained model that is configured by this machine learning method.

Finally, the signal removal unit 114 calculates the product of the basis matrix selected in the previous step (Step S250 and Step S250A in the examples illustrated in Fig. 9 and Fig. 10, respectively) and the weight matrix corresponding to the basis matrix (that is, reconstructs the DNA profile feature amount of the sample) and performs scaling (Step S260 in Figs. 9 and 10: a reconstruction step, and a reconstruction process). For the scaling, in the procedure described in Step S226 in the first embodiment, the basis matrix H' and the weight matrix W' are used instead of the residual matrix R (see Equations 1 and 2 and the like: the residual matrix E in the examples illustrated in Figs. 6 and 7), and the product of these matrices (reconstructed DNA profile feature amount) is divided by the tumor ratio (the abundance ratio of the tumor in the DNA profile feature amount) for each sample.

[Examples]

The effects of the embodiment of the present invention are shown on the basis of the following experimental conditions. In addition, the following experimental results are based on data of TCGA Research Network (https://www.cancer.gov/tcga).

[Experimental Conditions]

A DNA methylation state related to the following cancers acquired from TCGA was used as input data. The following samples in which the abundance ratio of the tumor was shown were selected from TCGA tumor data by Aran et al. [D. Aran, et al., 2015].

(1) Lung cancer (828 samples)
(2) Colorectal cancer (401 samples)
(3) Liver cancer (375 samples)
(4) Ovarian cancer (10 samples)

Further, the feature amount whose value was missing in all of the samples was removed, and about 390,000 dimensions were used.

The 1,614 samples were divided into samples for learning and samples for testing at a ratio of 50:50 such that classes were equal, and marker selection and classifier training were performed using learning data. For comparison, the following two types of learning and evaluation were performed.

1. Learning was performed with learning data before execution, and evaluation was performed with test data before execution.
2. Learning was performed with learning data after cleansing (data processing according to the embodiment of the present invention), and evaluation was performed with test data before cleansing.

[Results]

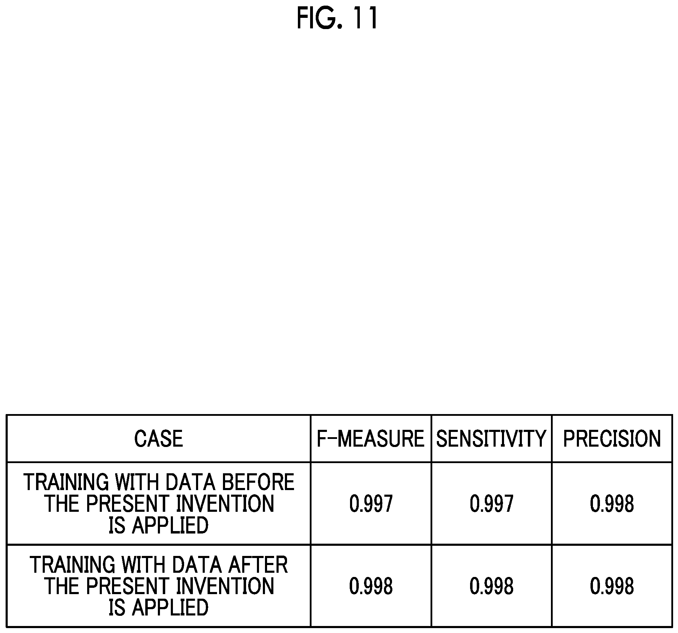

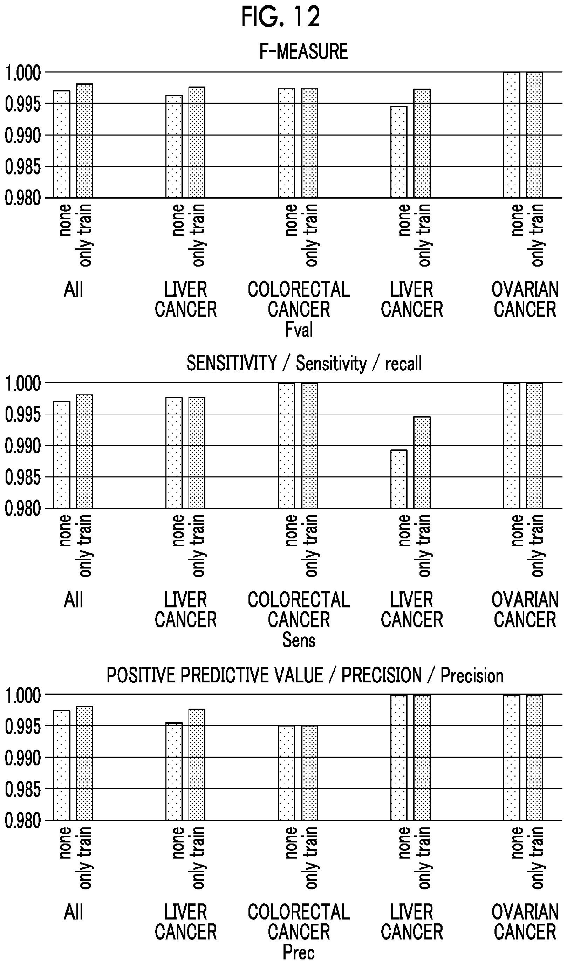

As a result, it could be confirmed that the performance of cancer multi-class classification prediction was improved by applying the data cleansing method (data processing method) according to the embodiment of the present invention (see Figs. 11 and 12 (tables and diagrams illustrating the results of prediction experiments)). In Fig. 12, "none" indicates the result before the present invention is executed, and "only train" indicates the result after the cleansing according to the embodiment of the present invention is executed. Here, sensitivity = (true positive)/(true positive + false negative), precision = (true positive)/(true positive + false positive), and F-measure = 2 × (sensitivity × precision)/(sensitivity + precision). The F-measure is the harmonic mean of the sensitivity and the precision. In addition, the "true positive" is a rate at which the cancer A is correctly predicted, the "false positive" is a rate at which the cancer A is erroneously predicted, and the "false negative" is a rate at which cancers other than the cancer A are erroneously predicted.

[Visualization of Results]

In addition, a change in the degree of methylation by the method (data processing method) according to the embodiment of the present invention was visualized. Fig. 13A illustrates a distribution of the degree of methylation of a methylation site included in a certain cancer sample (one of data items acquired by TCGA and used in the prediction experiment: see https://www.cancer.gov/tcga). In Fig. 13A, a horizontal axis indicates the degree of methylation (0 to 1) and a vertical axis indicates density. The distribution (displayed as "before") represented by a dotted line is a distribution before the application of this method, and it can be seen that there is a peak around 0.4 in addition to around 0 and around 1. Since the degree of methylation is, in principle, a binary value of 0 or 1, the former two peaks are appropriate. On the other hand, the peak observed around 0.4 strongly suggests that cells in a plurality of different methylation states are mixed in this sample. The distribution (displayed as "after") represented by a solid line is a distribution after this method is applied, and it can be seen that a peak having an intermediate value is reduced.
For comparison, in Fig. 13B, a case where this method is applied to a sample having a low non-cancer cell mixing ratio (= a sample having a high tumor ratio: see https://www.cancer.gov/tcga) was visualized.

Unlike the previous example, it can be seen that there is almost no change (difference between the distribution represented by "before" and the distribution represented by "after") in the degree of methylation by this method. The comparison shows that this method does not work unintentionally in a case where the mixture of non-cancer cells is not suspected.

The embodiment of the present invention has been described above. However, the present invention is not limited to the above-described aspects and can be modified in various ways without departing from the gist of the present invention.

Explanation of References

    10: data processing apparatus
    100: processing unit
    110: processor
    112: input unit
    114: signal removal unit
    116: output unit
    120: ROM
    130: RAM
    200: storage unit
    300: display unit
    310: monitor
    400: operation unit
    410: keyboard
    420: mouse
    500: external server
    510: external database
    700: first input data
    702: second input data
    704: typical pattern matrix
    710: output data
    E: residual matrix
    H': basis matrix
    N1: number of samples
    NW: network
    R: residual matrix
    W': weight matrix
    X0: basis
    X1: basis
    X2: basis
    X3: basis
    S100 to S300: steps of data processing method

**Claims**

1. A data processing method executed by a data processing apparatus including a processor, the data processing method comprising:
   causing the processor to execute:

   an input step of inputting first DNA profile data obtained by measuring a sample including a tumor cell and one or more types of known non-tumor cells;
   a signal removal step of removing a signal derived from the non-tumor cell, which is mixed in the input first DNA profile data, to acquire a signal derived from the tumor cell; and
   an output step of outputting the signal derived from the tumor cell as a DNA profile feature amount of the sample.

**2.** The data processing method according to claim 1,
wherein the processor is configured to acquire information that reflects features of a cell and/or a tissue defined by a sequence and/or modification of DNA as the first DNA profile data in the input step.

**3.** The data processing method according to claim 2,
wherein the processor is configured to acquire, as the information, a measured value of at least one of a methylation state of DNA, mutation information of DNA, or a gene expression level.

**4.** The data processing method according to any one of claims 1 to 3,
wherein the processor is configured to:

input second DNA profile data that is different from the first DNA profile data and is sorted for each of known non-tumor cell types, which are likely to be mixed in the first DNA profile data, in the input step; and
in the signal removal step,

create a typical pattern matrix composed of typical patterns of the non-tumor cell types on the basis of the second DNA profile data,
decompose the first DNA profile data into a signal for each of the non-tumor cell types using the first DNA profile data and the typical pattern matrix,

remove a true residual from a residual of a result of the decomposition to calculate a residual corresponding to the signal derived from the tumor cell, and
scale the calculated residual to acquire the signal derived from the tumor cell.

**5.** The data processing method according to claim 4,
wherein the processor is configured to:

in a case where M, N, and K are positive integers, input N samples for M feature amounts as the second DNA profile data for K cell types; and
create K types of M-dimensional typical pattern vectors from the input second DNA profile data and connect the K types of typical pattern vectors to create the typical pattern matrix of M rows and K columns.

**6.** The data processing method according to claim 4 or 5,
wherein the processor is configured to perform the decomposition using a linear regression method.

**7.** The data processing method according to claim 6,
wherein the processor is configured to use a least square method or a robust linear regression method as the linear regression method.

**8.** The data processing method according to claim 4 or 5,
wherein the processor is configured to perform the decomposition with a semi-reference-based method using some of the known typical pattern matrices.

**9.** The data processing method according to claim 6 or 7,
wherein the processor is configured to extract a residual of a result of regression performed by the linear regression method and to perform post-processing based on properties of the DNA profile feature amount on the extracted residual.

**10.** The data processing method according to claim 9,
wherein the processor is configured to divide the calculated residual by an abundance ratio of a tumor in the input first DNA profile data to perform the scaling.

**11.** The data processing method according to any one of claims 4 to 10,

wherein the processor is configured to: in the signal removal step,
factorize a matrix indicating the first DNA profile data into a mixing ratio matrix indicating a mixing ratio of cell types and a typical pattern matrix for the mixing ratio matrix to acquire the signal derived from the tumor cell; and
reconstruct the DNA profile feature amount from the acquired signal.

**12.** The data processing method according to claim 11,
wherein the processor is configured to perform the matrix factorization using a singular value decomposition method or a non-negative matrix factorization method.

**13.** The data processing method according to any one of claims 1 to 3,
wherein the processor is configured to, in the signal removal step, acquire the signal derived from the tumor cell using an abundance ratio of a tumor in the first DNA profile data which has been calculated by a method different from matrix factorization and to reconstruct the DNA profile feature amount from the acquired signal.

**14.** The data processing method according to claim 13,
wherein the processor is configured to acquire the signal derived from the tumor cell using a machine learning method.

**15.** The data processing method according to any one of claims 11 to 14,
wherein the processor is configured to:

reconstruct the DNA profile feature amount of the sample including a component of the signal derived from the tumor cell, using a mixing ratio matrix corresponding to the acquired signal derived from the tumor cell; and divide the reconstructed DNA profile feature amount by the abundance ratio of the tumor in the DNA profile feature amount to perform scaling.

**16.** A data processing apparatus comprising:

a processor,
wherein the processor is configured to execute:

an input process of inputting first DNA profile data obtained by measuring a sample including a tumor cell and one or more types of known non-tumor cells;
a signal removal process of removing a signal derived from the non-tumor cell, which is mixed in the input first DNA profile data, to acquire a signal derived from the tumor cell; and
an output process of outputting the signal derived from the tumor cell as a DNA profile feature amount of the sample.

**17.** A data processing program causing a computer to execute a data processing method,
wherein the data processing method includes:

an input step of inputting first DNA profile data obtained by measuring a sample including a tumor cell and one or more types of known non-tumor cells;
a signal removal step of removing a signal derived from the non-tumor cell, which is mixed in the input first DNA profile data, to acquire a signal derived from the tumor cell; and
an output step of outputting the signal derived from the tumor cell as a DNA profile feature amount of the sample.

**18.** A non-transitory computer-readable recording medium storing the program according to claim 17.

## FIG. 1

# FIG. 2

100

110

| INPUT UNIT | ~112 |

| SIGNAL REMOVAL UNIT | ~114 |

| OUTPUT UNIT | ~116 |

| ROM | ~120 |

| RAM | ~130 |

# FIG. 3

S100
⟨INPUT DATA 1: MIXED DATA⟩
700
FEATURE AMOUNT DIMENSIONS $M$ × SAMPLES $N_1$

S200
NON-TUMOR CELL REMOVAL STEP
ANY METHOD

S300
⟨OUTPUT DATA 1: PURE TUMOR DATA⟩
710
$M$ × $N_1$

# FIG. 4

EP 4 411 741 A1

FIG. 5A

FIG. 5B

| #1 | |
|----|---|
| #2 | |
| #3 | |
| #4 | |
| #5 | |
| #6 | |
| #7 | |
| #8 | |

$= 0.4 \times$

| #1 | |
|----|---|
| #2 | |
| #3 | |
| #4 | |
| #5 | |
| #6 | |
| #7 | |
| #8 | |

\<measured\>
*X*

\<REFERENCE\>
*B*

*F* DESIRED TO BE CALCULATED
(NON-CANCER CELL MIXING RATIO)

↓ EACH POINT IS FEATURE
AMOUNTS #1 TO #8

Slope: 1.0

Slope: 0.4

*X*: measured

0.4

*B*: ref

1.0

MARKER AFFECTED
BY INFLUENCE OF TUMOR CELL WITH
ABUNDANCE RATIO OF 60% IS OUTLIER

EP 4 411 741 A1

# FIG. 6

SIGNAL DECOMPOSITION STEP

S220

REFERENCE-BASED DECOMPOSITION (S224)

700

FEATURE AMOUNT DIMENSIONS $M$ × SAMPLES $N_1$

FEATURE AMOUNT DIMENSION ($M$)

SAMPLE

CELL TYPE ($K$)

≈

FEATURE AMOUNT DIMENSION ($M_{partial}$)

CELL TYPE ($K$)

⟨MIXED KNOWN CELL TYPE REFERENCE: $H'$⟩

×

CELL TYPE

SAMPLE ($N_1$)

?

⟨WEIGHT MATRIX: $W$⟩

+

FEATURE AMOUNT DIMENSION ($M$)

?

⟨RESIDUAL MATRIX: $E$⟩

MARKER SELECTION (S222)

RECONSTRUCTION ERROR CALCULATION (S226)

704

FEATURE AMOUNT DIMENSIONS $M$ × CELL TYPE $K$

FEATURE AMOUNT DIMENSION ($M$)

CELL TYPE

⟨MIXED KNOWN CELL TYPE REFERENCE: $H$⟩

SELECT ONLY MARKER HAVING VALUE VARYING BETWEEN MIXED KNOWN CELL TYPES (S222)

→ IMPROVEMENT OF DECOMPOSITION ACCURACY
+ REDUCTION IN CALCULATION COST

FEATURE AMOUNT DIMENSION ($M$)

SAMPLE

−

FEATURE AMOUNT DIMENSION ($M$)

CELL TYPE

×

CELL TYPE

SAMPLE ($N_1$)

⟨WEIGHT MATRIX: $W$⟩

EP 4 411 741 A1

# FIG. 7

PERFORM DECOMPOSITION CONSIDERING THAT
"THERE IS NO CANCER CELL (ONLY ASSUMED CANCER CELL IS MIXED)"
⇒ REFERENCE-BASED MATRIX DECOMPOSITION

REFERENCE-BASED DECOMPOSITION (S224)

⟨INPUT DATA: $D$⟩

SAMPLE

FEATURE AMOUNT DIMENSION (TO 4 MILLION SITES)

≈

NON-CANCER CELL TYPE

FEATURE AMOUNT DIMENSION (TO 4 MILLION SITES)

⟨REFERENCE (TYPICAL PATTERN): $H$⟩

×

SAMPLE

NON-CANCER CELL TYPE

?

⟨WEIGHT MATRIX: $W$⟩

+

SAMPLE

FEATURE AMOUNT DIMENSION (TO 4 MILLION SITES)

⟨RESIDUAL MATRIX: $E$⟩
⇒ PURE DATA

COMPONENT THAT IS NOT ADDITION OF NON-CANCER CELLS
→ CANCER CELL IS MIXED
→ CANCER-DERIVED SIGNAL IS EXTRACTED TO RESIDUAL MATRIX

EP 4 411 741 A1

# FIG. 8

S200B

NON-TUMOR CELL REMOVAL STEP

S100

⟨INPUT DATA 1: MIXED DATA⟩

700

FEATURE AMOUNT DIMENSIONS $M$ × SAMPLES $N_1$

S222

SIGNAL DECOMPOSITION STEP

DECOMPOSE DATA INTO SIGNALS FOR EACH CELL TYPE: REFERENCE-FREE

S250

TUMOR-DERIVED SIGNAL EXTRACTION STEP

(1) USING TUMOR RATIO
(2) SUPERVISED LEARNING

S260

RECONSTRUCTION STEP

ESTIMATE TUMOR RATIO OF SAMPLE

S300

⟨OUTPUT DATA⟩

710

$M × N_1$

# FIG. 9

SIGNAL DECOMPOSITION STEP    REFERENCE-FREE DECOMPOSITION    S222

700

FEATURE AMOUNT DIMENSIONS $M \times$ SAMPLES $N_1$

SAMPLE $N_1$ / FEATURE AMOUNT DIMENSION ($M$)

$\approx$

BASIS ($K$) / FEATURE AMOUNT DIMENSION ($M$)

$\times$

SAMPLE ($N_1$) / BASIS ⟨WEIGHT MATRIX: $W$⟩

$+$

FEATURE AMOUNT DIMENSION ($M$) ⟨RESIDUAL MATRIX: $E$⟩

RECONSTRUCTION STEP    S260

$H' \cdot W'$ /TUMOR RATIO
CALCULATE PRODUCT OF BASIS MATRIX FROM WHICH NON-CANCER COMPONENT HAS BEEN REMOVED AND WEIGHT MATRIX AND DIVIDE PRODUCT BY TUMOR RATIO FOR EACH SAMPLE.

S250    TUMOR-DERIVED SIGNAL EXTRACTION STEP

SAMPLE ($N_1$)

| BASIS | | | | | | |
|---|---|---|---|---|---|---|
| X3 | 0.8 | 0.9 | 0.7 | 0.1 | 0.1 | 0.0 |
| X2 | 0.1 | 0.1 | 0.0 | 0.8 | 0.9 | 0.7 |
| X1 | 0.8 | 0.9 | 0.7 | 0.8 | 0.9 | 0.7 |
| X0 | 0.4 | 0.3 | 0.2 | 0.3 | 0.4 | 0.3 |

USING TUMOR RATIO

TUMOR RATIO / WEIGHT FOR BASIS

X1 + X2 + X3 POSITIVELY CORRELATES WITH TUMOR RATIO
⇒ ESTIMATED AS CANCER-DERIVED SIGNAL

X0: DELETE NON-CANCER BASIS

X0 NEGATIVELY CORRELATES WITH TUMOR RATIO ⇒ ESTIMATED AS NON-CANCER-DERIVED SIGNAL

FEATURE AMOUNT DIMENSION ($M$)

X1  X2  X3

⟨BASIS MATRIX: $H'$⟩

SAMPLE ($N_1$)

| BASIS | | | | | | |
|---|---|---|---|---|---|---|
| X3 | 0.8 | 0.9 | 0.7 | 0.1 | 0.1 | 0.0 |
| X2 | 0.1 | 0.1 | 0.0 | 0.8 | 0.9 | 0.7 |
| X1 | 0.8 | 0.9 | 0.7 | 0.8 | 0.9 | 0.7 |

⟨WEIGHT MATRIX: $W'$⟩

EP 4 411 741 A1

# FIG. 10

SIGNAL DECOMPOSITION STEP    REFERENCE-FREE DECOMPOSITION    S222    RECONSTRUCTION STEP    S260

700

FEATURE AMOUNT DIMENSIONS M × SAMPLES $N_i$

FEATURE AMOUNT DIMENSION (M)    SAMPLE $N_i$    ≈    FEATURE AMOUNT DIMENSION (M)    BASIS (K)    ×    BASIS    SAMPLE ($N_i$)    ?    〈WEIGHT MATRIX: W〉    +    FEATURE AMOUNT DIMENSION (M)    ?    〈RESIDUAL MATRIX: E〉

H'· W' /TUMOR RATIO
CALCULATE PRODUCT OF BASIS MATRIX FROM WHICH NON-CANCER COMPONENT HAS BEEN REMOVED AND WEIGHT MATRIX AND DIVIDE PRODUCT BY TUMOR RATIO FOR EACH SAMPLE.

S250A    TUMOR-DERIVED SIGNAL EXTRACTION STEP

SAMPLE ($N_i$)

| BASIS | | | | | | |
|---|---|---|---|---|---|---|
| X3 | 0.8 | 0.9 | 0.7 | 0.1 | 0.1 | 0.0 |
| X2 | 0.1 | 0.1 | 0.0 | 0.8 | 0.9 | 0.7 |
| X1 | 0.8 | 0.9 | 0.7 | 0.8 | 0.9 | 0.7 |
| X0 | 0.4 | 0.3 | 0.2 | 0.3 | 0.4 | 0.3 |

CANCER TYPE 1    CANCER TYPE 2

SINCE ESTIMATION RATIO IS HIGH IN SAMPLE OF CANCER TYPE 1, CORRESPONDENCE THAT "X3 IS CANCER TYPE 1" CAN BE PRESUMED. (THE SAME IS APPLIED TO X2)

X0: NON-CANCER BASIS IS DELETED

SINCE VALUE IS HIGH IN COMMON FOR ANY TYPE, BASIS IS COMMON TO CANCER TYPE

SINCE VALUE IS LOW IN COMMON FOR ANY TYPE, BASIS IS ESTIMATED AS BASIS DERIVED FROM NON-CANCER CELL

USING CANCER TYPE LABEL

FEATURE AMOUNT DIMENSION (M)    X1 X2 X3

〈BASIS MATRIX: H'〉

SAMPLE ($N_i$)

| BASIS | | | | | | |
|---|---|---|---|---|---|---|
| X3 | 0.8 | 0.9 | 0.7 | 0.1 | 0.1 | 0.0 |
| X2 | 0.1 | 0.1 | 0.0 | 0.8 | 0.9 | 0.7 |
| X1 | 0.8 | 0.9 | 0.7 | 0.8 | 0.9 | 0.7 |

CANCER TYPE 1    CANCER TYPE 2

〈WEIGHT MATRIX: W'〉

EP 4 411 741 A1

# FIG. 11

| CASE | F-MEASURE | SENSITIVITY | PRECISION |
|---|---|---|---|
| TRAINING WITH DATA BEFORE THE PRESENT INVENTION IS APPLIED | 0.997 | 0.997 | 0.998 |
| TRAINING WITH DATA AFTER THE PRESENT INVENTION IS APPLIED | 0.998 | 0.998 | 0.998 |

## FIG. 12

**F-MEASURE**

**SENSITIVITY / Sensitivity / recall**

**POSITIVE PREDICTIVE VALUE / PRECISION / Precision**

## FIG. 13A

LUNG CANCER
TUMOR RATIO 63%

after

before

Density

Tumor_LUAD_TCGA-05-4427-01A-21D-1856-05

## FIG. 13B

COLORECTAL CANCER
TUMOR RATIO 91%

after

before

Density

Tumor_COAD_TCGA-NH-A50T-01A-11D-A28O-05

| INTERNATIONAL SEARCH REPORT | International application No. |
|---|---|
| | **PCT/JP2022/029057** |

**A.    CLASSIFICATION OF SUBJECT MATTER**

*G16B 40/00*(2019.01)i
FI:   G16B40/00

According to International Patent Classification (IPC) or to both national classification and IPC

**B.    FIELDS SEARCHED**

Minimum documentation searched (classification system followed by classification symbols)

G16B 5/00 - 99/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | QIN, Yufang et al. Deconvolution of heterogeneous tumor sanples using partial reference signals, [online]. 30 December 2020, pp. 1-18, [retrieval date:16 August 2022], <URL:https://doi.org/10.1371/journal.pcbi.1008452> | 1-3, 16-18 |
| | pp. 3, 6-8 | |
| Y | pp. 3, 6-8 | 13-15 |
| A | entire text, all drawings | 4-12 |
| Y | ZAYTCREV, Alexander et al. Novel machine learning based deconvolution algorithm results in accurate description of tumor microenvironment from bulk RNAseq. Canser Research. 15 August 2020, vol. 80, issue 16, pp. 1-2, [retrieval date:16 August 2022], <URL:https://aacrjournals.org/cancerres/article/80/16_Supplement/853/644999/Abstract-853-Novel-machine-learning-based> | 13-15 |
| | abstract | |
| A | JP 2009-537827 A (UNIVERSITY OF UTAH RESEARCH FOUNDATION) 29 October 2009 (2009-10-29) | 1-18 |
| | entire text, all drawings | |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 August 2022** | **30 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

| International application No. |
| --- |
| **PCT/JP2022/029057** |

| Patent document<br>cited in search report | Publication date<br>(day/month/year) | Patent family member(s) | Publication date<br>(day/month/year) |
| --- | --- | --- | --- |
| JP 2009-537827 A | 29 October 2009 | US 2007/0269837 A1<br>entire text, all drawings<br>WO 2007/137124 A2<br>EP 2021802 A2<br>CA 2652382 A1<br>AU 2007253771 A1 | |

Form PCT/ISA/210 (patent family annex) (January 2015)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- JP 2009537827 A

## Non-patent literature cited in the description

- **LI WANG et al.** *A reference profile-free deconvolution method to infer cancer cell-intrinsic subtypes and tumor-type-specific stromal profiles,* 22 September 2021, https://genomemedicine.biomedcentral.com/track/pdf/10.1186/s13073-020-0720-0.pdf
- **DOUGLAS ARNESON et al.** *MethylResolver-a method for deconvoluting bulk DNA methylation profiles into known and unknown cell contents,* 22 September 2021, https://www.nature.com/articles/s42003-020-01146-2.pdf
- **ROUSSEEUW, P. J. ; LEROY, A. M.** Robust Regression and Outlier Detection. Wiley, 1987
- **DOUGLAS ARNESON et al.** *MethylResolver-a method for deconvoluting bulk DNA methylation profiles into known and unknown cell contents,* 22 September 2021, https://www.nature.com /articles/s42003-020-01146-2.pdf
- **S. L. CARTER et al.** *Absolute quantification of somatic DNA alterations in human cancer,* 2012, https://dash.harvard.edu/handle/1/15034760
- **K. YOSHIHARA et al.** *Inferring tumour purity and stromal and immune cell admixture from expression data,* 2013, https://www.nature.com/articles/ncomms3612.pdf